(19) Europäisches Patentamt
European Patent Office
Office européen des brevets

(11) Veröffentlichungsnummer : **0 472 497 A1**

(12) **EUROPÄISCHE PATENTANMELDUNG**

(21) Anmeldenummer : **91810640.2**

(51) Int. Cl.$^5$ : **C07C 327/22,** A01N 37/02

(22) Anmeldetag : **13.08.91**

(30) Priorität : **21.08.90 CH 2708/90**

(43) Veröffentlichungstag der Anmeldung :
**26.02.92 Patentblatt 92/09**

(84) Benannte Vertragsstaaten :
**BE CH DE DK ES FR GB GR IT LI LU NL**

(71) Anmelder : **CIBA-GEIGY AG**
**Klybeckstrasse 141**
**CH-4002 Basel (CH)**

(72) Erfinder : **Maienfisch, Peter, Dr.**
**Aegertenstrasse 21**
**CH-4118 Rodersdorf (CH)**
Erfinder : **Böger, Manfred, Dr.**
**Wilhelm Glockstrasse 14**
**W-7858 Weil am Rhein 5 (DE)**
Erfinder : **Steiner, Eginhard, Dr.**
**Obere Hofackerstrasse 3**
**CH-4414 Füllinsdorf (DE)**

(54) **Thiobuttersäurederivate.**

(57) Neue 4-Chlor-4,4-difluorthiobuttersäure-Derivate Formel I

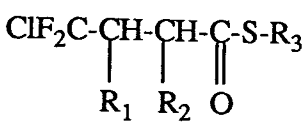

(I)

worin
$R_1$ und $R_2$ unabhängig voneinander Wasserstoff, $C_1$-$C_4$-Alkyl oder $C_1$-$C_4$-Halogenalkyl und
$R_3$ Wasserstoff oder einen organischen Rest bedeuten, können als Schädlingsbekämpfungsmittel eingesetzt werden. Vorzugsweise können Insekten und Arachniden bekämpft werden.

EP 0 472 497 A1

Jouve, 18, rue Saint-Denis, 75001 PARIS

Die vorliegende Erfindung betrifft neue Derivate von 4-Chlor-4,4-difluorthiobuttersäure, Verfahren und Zwischenprodukte zu ihrer Herstellung, Schädlingsbekämpfungsmittel, welche diese Verbindungen enthalten, sowie ihre Verwendung bei der Kontrolle von Schädlingen.

Die erfindungsgemässen 4-Chlor-4,4-difluorthiobuttersäurederivate entsprechen der Formel I

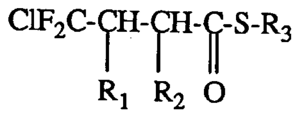

(I)

Worin
$R_1$ und $R_2$ unabhängig voneinander Wasserstoff, $C_1$-$C_4$-Alkyl oder $C_1$-$C_4$-Halogenalkyl, und
$R_3$ Wasserstoff oder einen organischen Rest bedeuten.

Aus der Literatur ist die Klasse der polyhalogenierten Buttersäurechloride als Zwischenprodukte für pyrethroide Haloketone aus Helv. Chim. Acta 63, p. 1947-1957 ( 1980) bekannt.

Der in der Definition von $R_3$ genannte organischen Rest steht für ein beliebiges organisches Radikal, das in Form eines Mercaptans an die Carbonylgruppe der 4-Chlor-4,4-difluorbuttersäure gebunden werden kann. Dabei ist die Mercaptanfunktion dieses organischen Radikals an ein Kohlenstoffatom gebunden. Damit wird der Rest $R_3$ vorzugsweise über ein Kohlenstoffatom an die -CO-S-Gruppe gebunden. Beispielsweise steht für $R_3$ jeweils substituiertes oder unsubstituiertes $C_1$-$C_{20}$-Alkyl, $C_3$-$C_6$-Cycloalkyl, $C_3$-$C_{10}$-Alkenyl, Benzyl oder Aryl. Vorzugsweise steht $R_3$ im Rahmen der vorliegenden Erfindung für $C_1$-$C_{20}$-Alkyl, $C_3$-$C_6$-Cycloalkyl, $C_3$-$C_{10}$-Alkenyl, Aryl, $C_3$-$C_{10}$-Halogenalkenyl; durch Halogen substituiertes $C_3$-$C_6$-Cycloalkyl; durch Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_6$-Alkoxycarbonyl, $C_1$-$C_4$-Alkylthio oder Nitro, substituiertes Aryl; oder durch Halogen, Di-$C_1$ -$C_4$-alkylamino, $C_1$-$C_4$-Alkoxy, $C_1$-$C_4$-Alkylthio, $C_1$-$C_{20}$-Alkoxycarbonyl, $C_3$-$C_7$-Cycloalkoxycarbonyl, $C_1$-$C_4$-Alkylcarbonyloxy, $C_3$-$C_6$-Cycloalkyl, Aryl, $C_1$-$C_{10}$-Alkylaminocarbonyl oder Anilinocarbonyl, substituiertes $C_1$-$C_{20}$-Alkyl; wobei die Arylgruppen jeweils durch Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder Nitro substituiert sein können.

In der Definition der erfindungsgemässen Formel I sollen die einzelnen generischen Begriffe wie folgt verstanden werden:

Bei den als Substituenten in Betracht kommenden Halogenatomen handelt es sich sowohl um Fluor und Chlor als auch um Brom und Jod, wobei Fluor, Chlor und Brom bevorzugt sind. Halogen ist dabei als selbständiger Substituent oder als Teil eines Substituenten zu verstehen wie im Halogenalkyl, Halogenalkoxy oder Halogenphenoxy.

Die als Substituenten in Betracht kommenden Alkyl-, Alkylthio- und Alkoxyreste können geradkettig oder verzweigt sein. Als Beispiele solcher Alkyle seien Methyl, Aethyl, Propyl, Isopropyl, Butyl, i-Butyl, sek.Butyl, tert.Butyl oder Pentyl, Hexyl, Octyl, Decyl, Dodecyl und ihre Isomeren genannt. Als geeignete Alkoxyreste seien unter anderem genannt: Methoxy, Aethoxy, Propoxy, Isopropoxy oder Butoxy und ihre Isomeren. Alkylthio steht beispielsweise für Methylthio, Aethylthio, Isopropylthio, Propylthio oder die isomeren Butylthio.

Sind die als Substituenten in Betracht kommenden Alkyl-, Alkoxy-, Alkenyl- oder Arylgruppen durch Halogen substituiert, so können sie nur teilweise oder auch perhalogeniert sein. Dabei gelten für Halogen, Alkyl und Alkoxy die oben gegebenen Definitionen. Beispiele der Alkylelemente dieser Gruppen sind das ein- bis dreifach durch Fluor, Chlor und/oder Brom substituierte Methyl wie beispielsweise $CHF_2$ oder $CF_3$; das ein- bis fünffach durch Fluor, Chlor und/oder Brom substituierte Aethyl wie zum Beispiel $CH_2CF_3$, $CF_2CF_3$, $CF_2CCl_3$, $CF_2CHCl_2$, $CF_2CHF_2$, $CF_2CFCl_2$, $CF_2CHBr_2$, $CF_2CHClF$, $CF_2CHBrF$ oder $CClFCHClF$; das ein- bis siebenfach durch Fluor, Chlor und/oder Brom substituierte Propyl oder Isopropyl wie z.B. $CH_2CHBrCH_2Br$, $CF_2CHFCF_3$, $CH_2CF_2CF_3$ oder $CH(CF_3)_2$; das ein- bis neunfach durch Fluor, Chlor und/oder Brom substituierte Butyl oder eines seiner Isomeren wie zum Beispiel $CF(CF_3)CHFCF_3$ oder $CH_2(CF_2)_2CF_3$.

Sind die unter $R_3$ definierten Alkyl-, Cycloalkyl- oder Arylgruppen durch andere Substituenten substituiert, so können sie ein- oder mehrfach durch den gleichen oder verschiedene der aufgezählten Substituenten substituiert sein. Vorzugsweise sind in dem substituierten Gruppen ein oder zwei weitere Substituenten vorhanden.

Bei den als Substituenten in Betracht kommenden Cycloalkylresten handelt es sich beispielsweise um Cyclopropyl, Cyclobutyl, Cyclopentyl oder Cyclohexyl.

Alkenylgruppen enthalten eine oder mehrere, vorzugsweise nicht mehr als drei, ungesättigte Kohlenstoff-Kohlenstoff-Bindungen. Die Doppelbindungen sind von der Verknüpfungsstelle zum Schwefelatom durch mindestens ein gesättigtes Kohlenstoffatom getrennt. Typische Vertreter sind Allyl, Methallyl, 2-Butenyl oder 3-Butenyl.

Aryl steht für einen aromatischen Kohlenwasserstoffrest oder einen aromatischen, heterocyclischen Rest wie Furanyl oder Thienyl. Vorzugsweise wird unter Aryl Phenyl oder Naphthyl verstanden.

Beispiele für Alkoxycarbonylreste sind Methoxycarbonyl, Aethoxycarbonyl, Propoxycarbonyl, Isopropoxycarbonyl oder Butoxycarbonyl. Alkylcarbonyl steht beispielsweise für Acetyl, Propionyl, Butyryl oder Valeryl, sowie Isomere davon. Alkylcarbonyloxy steht zum Beispiel für Acetoxy, Propionyloxy oder Butyryloxy.

Beispiele für Cycloalkoxycarbonyl sind Cyclopropoxycarbonyl, Cyclopentyloxycarbonyl, Cyclohexyloxycarbonyl oder Cycloheptyloxycarbonyl.

Arylgruppen, insbesondere Phenylkerne können weitere Substituenten tragen, beispielsweise je bis zu drei Reste aus der Gruppe Halogen, Alkyl, Halogenalkyl und Alkoxy oder je ein oder zwei Substituenten aus der Gruppe Nitro, Halogenalkoxy, Alkylthio und Cycloalkyl. Insgesamt ist die Anzahl der Substituenten am Phenylring zusammen in der Regel nicht grösser als vier. Vorzugsweise tragen diese Phenylgruppen nicht mehr als 3 Substituenten aus der Reihe Chlor, Brom, Methyl, Aethyl und Trifluormethyl.

Unter den Verbindungen der Formel I sind solche Untergruppen herauszuheben, in denen entweder

a) $R_1$ und $R_2$ unabhängig voneinander Wasserstoff bedeuten, oder

b) $R_3$ für $C_1$-$C_{20}$-Alkyl, $C_3$-$C_6$-Cycloalkyl, $C_3$-$C_{10}$-Alkenyl, Phenyl, Naphthyl, $C_3$-$C_{10}$-Halogenalkenyl; durch Fluor, Chlor oder Brom substituiertes $C_3$-$C_6$-Cycloalkyl; durch Fluor, Chlor, Brom, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_4$-Alkoxycarbonyl, $C_1$-$C_3$-Alkylthio oder Nitro, substituiertes Phenyl, oder durch Fluor, Chlor, Brom, Di-$C_1$-$C_4$-alkylamino, $C_1$-$C_4$-Alkoxy, $C_1$-$C_4$-Alkylthio, $C_1$-$C_{20}$-Alkoxycarbonyl, $C_3$-$C_7$-Cycloalkoxycarbonyl, $C_1$-$C_4$-Alkylcarbonyloxy, $C_3$-$C_6$-Cycloalkyl, Phenyl, $C_1$-$C_{10}$-Akylaminocarbonyl oder Anilinocarbonyl substituiertes $C_1$-$C_{20}$-Alkyl, wobei die Phenylgruppen jeweils durch Fluor, Chlor, Brom, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio oder Nitro substituiert sein können, steht.

Aus der Untergruppe b) der Verbindungen der Formel I sind einerseits diejenigen herauszustellen, worin $R_3$ Phenyl, Benzyl, oder jeweils durch Fluor, Chlor, Brom, $C_1$-$C_3$-Alkyl, $C_1$-$C_3$-Halogenalkyl, $C_1$-$C_3$-Alkoxy, $C_1$-$C_4$-Alkoxycarbonyl, $C_1$-$C_3$-Halogenalkoxy, $C_1$-$C_3$-Alkylthio oder Nitro, substituiertes Phenyl oder Benzyl bedeutet. Von diesen Verbindungen sind wiederum solche bevorzugt, worin $R_1$ und $R_2$ unabhängig voneinander Wasserstoff bedeuten.

Andererseits sind unter der Untergruppe b) der Verbindungen der Formel I auch diejenigen besonders hervorzuheben, worin $R_3$ $C_1$-$C_{12}$-Akyl oder durch Hydroxy, Fluor, Chlor, Brom, Dimethylamino, Methoxy, Aethoxy, Methylthio, Aethylthio, Cyclopropyl, Cyclopentyl, Cyclohexyl oder Phenyl substituiertes $C_1$-$C_{12}$-Alkyl bedeutet, wobei der Phenylrest jeweils durch Fluor, Chlor, Brom, Methyl, Methoxy, Trifluormethyl, Trifluormethoxy oder Nitro substituiert sein kann. Von dieser Gruppe von Verbindungen sind solche bevorzugt, worin $R_1$ und $R_2$ unabhängig voneinander Wasserstoff bedeuten.

Als bevorzugte Einzelverbindungen der Formel I sind zu nennen:

4-Chlor-4,4-difluorthiobuttersäure-S-benzylester,
4-Chlor-4,4-difluorthiobuttersäure-S-phenylester,
4-Chlor-4,4-difluorthiobuttersäure-S-(4-toluyl)-ester und
4-Chlor-4,4-difluorthiobuttersäure-S-(3-chlor-phenyl)-ester.

Die erfindungsgemässen Verbindungen der Formel I können in Analogie zu bekannten Verfahren hergestellt werden. Beispielsweise erhält man die Verbindung der Formel I, indem man entweder

a) ein 4-Chlor-4,4-difluorbuttersäurehalogenid der Formel II

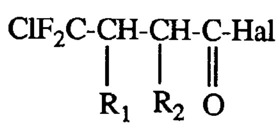

(II)

worin $R_1$ und $R_2$ die unter Formel I gegebenen Bedeutungen haben und Hal für Halogen, vorzugsweise Chlor oder Brom steht, vorzugsweise in Gegenwart einer Base mit einem Mercaptan der Formel III

$$H\text{-}S\text{-}R_3 \quad \text{(III)}$$

worin $R_3$ die unter Formel I gegebene Bedeutung hat, umsetzt, oder

b) eine 4-Chlor-4,4-difluorbuttersäure der Formel IV

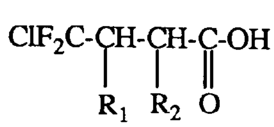

(IV)

worin $R_1$ und $R_2$ die unter Formel I gegebenen Bedeutungen haben, in Gegenwart eines wasserabspaltenden Mittels mit einem Mercaptan der Formel III umsetzt.

Die Umsetzung des Verfahrens a) (II+III → I) erfolgt vorzugsweise in einem inerten, hydroxylgruppenfreien Lösungsmittel in Anwesenheit einer organischen Base, wie zum Beispiel einem Amin wie Pyridin, 4-Dimethylaminopyridin, Lutidin, Collidin, Trialkylamin, N,N-Dialkylanilin, oder einer bicyclischen, nicht nucleophilen Base wie 1,4-Diazabicyclo[2.2.2]octan (DABCO), 1,5-Diazabicyclo[4.3.0]non-5-en (DBN) oder 1,8-Diazabicyclo[5.4.0]undec-7-en (1,5-5) (DBU). Die Reaktion wird im allgemeinen bei Temperaturen von -30° bis + 120°C, vorzugsweise von - 10° bis +80°C durchgeführt. Man arbeitet dabei zweckmässigerweise in Gegenwart eines reaktionsinerten Lösungsmittels oder Lösungsmittelgemisches. Es eignen sich hierfür beispielsweise aliphatische und aromatische Kohlenwasserstoffe wie Benzol, Toluol, Xylole, Petroläther, Hexan; halogenierte Kohlenwasserstoffe wie Chlorbenzol, Methylenchlorid, Aethylenchlorid, Chloroform, Tetrachlorkohlenwasserstoff, Tetrachloräthylen; Aether und ätherartige Verbindungen wie Dialkyläther (Diäthyläther, Düsopropyläther, tert.-Butylmethyläther usw.), Anisol, Dioxan, Tetrahydrofuran; Nitrile wie Acetonitril, Propionitril; Ester wie Aethylacetat (Essigsäureäthylester), Propylacetat oder Butylacetat; Ketone wie Aceton, Diäthylketon, Methyläthylketon; Verbindungen wie Dimethylsulfoxid (DMSO), Dimethylformamid (DMF) und Gemische solcher Lösungsmittel untereinander. Man kann die Reaktion aber auch im Ueberschuss einer der oben genannten Basen durchführen.

Bei der Verfahrensvariante b) (IV + III → I) wird die Reaktion vorteilhafterweise in Gegenwart von für Veresterungen von Thiocarbonsäuren üblichen, wasserabspaltenden und die Carbonylfunktion aktivierenden Reagenzien durchgeführt, wie zum Beispiel in Anwesenheit eines phosphorhaltigen Säurederivates wie Cyanphosphonsäure-diäthylester, Azidophosphorsäurediphenylester, Chlorphosphorsäure-diäthylester oder Diphenylphosphinsäure-chlorid, eines Harnstoffderivats wie N,N′-Carbonyldiimidozol oder N,N′-Carbonyldi-1,2,4-triazol oder eines 1-Alkyl-2-halogen-pyridiniumsalzes wie 1-Methyl-2-chlorpyridiniumjodid oder 1-Methyl-2-fluorpyridiniumjodid. Man arbeitet dabei zweckmässigerweise in Gegenwart eines reaktionsinerten Lösungsmittels oder Lösungsmittelgemisches bei Temperaturen von -30°C bis +120°C, vorzugsweise -10°C bis +80°C. Man arbeitet häufig auch in Gegenwart einer Base wie beispielsweise in Gegenwart eines organischen Amins wie eines Trialkylamins (Trimethylamin, Triäthylamin, Tripropylamin oder Diisopropyläthylamin), eines Pyridins (Pyridin selbst, 4-Dimethylaminopyridin oder 4-Pyrrolidinopyridin), eines Morpholins (N-Methylmorpholin) oder eines N,N-Dialkylanilins (N,N-Dimethylanilin oder N-Methyl-N-äthylanilin) oder einer in der Variante a) genannten Basen. Als Lösungsmittel eignen sich beispielsweise aliphatische und aromatische Kohlenwasserstoffe wie Benzol, Toluol, Xylole, Petroläther, Hexan; halogenierte Kohlenwasserstoffe wie Chlorbenzol, Methylenchlorid, Aethylenchlorid, Chloroform, Tetrachlorkohlenstoff, Tetrachloräthylen; Aether und ätherartige Verbindungen wie Dialkyläther (Diäthyläther, Diisopropyläther), tert.-Butylmethyläther usw.), Anisol, Dioxan, Tetrahydrofuran; Nitrile wie Acetonitril, Propionitril; Ester wie Aethylacetat (Essigsäureäthylester), Propylacetat oder Butylacetat; und Gemische solcher Lösungsmittel untereinander.

Grundsätzlich sind die verschiedenen Derivate der Formel I auch aus Umesterung aus den leicht zugänglichen Niederalkylestern der 4-Chlor-4,4-difluorbuttersäure erhältlich.

Beispielsweise können die Derivate der Formel I durch basenkatalysierte Umesterung der Niederalkylester der Formel V

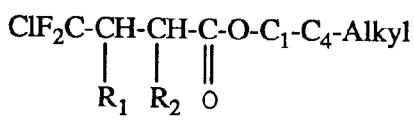

(V)

mit den Mercaptanen der Formel III

$$H\text{-}S\text{-}R_3 \qquad \text{(III)}$$

worin $R_3$ die unter Formel I gegebene Bedeutung hat, erhalten werden. Bei der basenkatalysierten Umesterung verwendet man vorzugsweise als Base das Natrium- oder Kaliumsalze des Mercaptans der Formel III, welche aus III beispielsweise durch Zugabe von Natrium- oder Kaliumhydrid zugänglich sind. Die Umesterungsreaktion wird vorzugsweise bei Temperaturen zwischen -20°C und +120°C, insbesondere zwischen - 10°C und +80°C durchgeführt. Die Mercaptankomponente III wird mit Vorteil im Ueberschuss verwendet. Als Lösungsmittel kommen Aether, wie Diäthyläther, Diisopropyläther, Dioxan oder Tetrahydrofuran, halogenierte Kohlenwasserstoffe oder aliphatische oder aromatische Kohlenwasserstoffe in Frage.

Die Verbindungen der Formel III sind bekannt und teilweise im Handel erhältlich oder sie können analog zu bekannten Herstellungsverfahren hergestellt werden.

Die Säurehalogenide der Formel II können auf übliche Weise durch Umsetzung mit Halogenierungsmitteln aus den 4-Chlor-4,4-difluorbuttersäuren der Formel IV erhalten werden. Als Halogenierungsmittel eignen sich besonders $SOCl_2$, Oxalylchlorid, $PCl_3$, $POCl_3$ oder $PCl_5$. Man arbeitet dabei im allgemeinen bei Temperaturen zwischen -20°C und +120°C, vorzugsweise zwischen 0°C und +100°C. Die Umsetzung kann ohne Lösungsmittel oder im Gemisch mit einem reaktionsinerten Lösungsmittel erfolgen. Als Lösungsmittel eignen sich hierfür beispielsweise aromatische Kohlenwasserstoff wie Benzol oder Toluol oder halogenierte Kohlenwasserstoffe wie Methylenchlorid, Chloroform oder Chlorbenzol. Die Reaktion wird häufig unter Zugabe einer katalytischen Menge von DMF durchgeführt.

Die 4-Chlor-4,4-difluorbuttersäuren der Formel IV lassen sich aus Verbindungen der Formel V durch saure oder basische Hydrolyse erhalten.

Die Verbindungen der Formel V sind nach dem folgenden Verfarehn erhältlich:

Die Verbindungen der Formel V können erhalten werden, indem man einen α-Halogen-4-chlor-4,4-difluorbuttersäureester der Formel

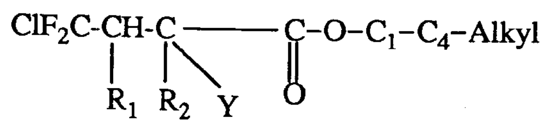

worin $R_1$ und $R_2$ die unter Formel I gegebenen Bedeutungen haben und Y für Chlor oder Brom steht, mit Wasserstoff in der α-Position katalytisch dehalogeniert.

Die Verbindungen der engeren Formel

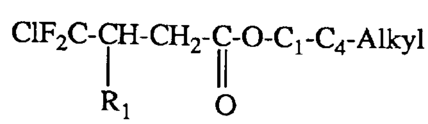

worin $R_1$ die unter Formel I gegebene Bedeutung hat, können durch katalytische α-Dehalogenierung mit Wasserstoff aus den α,α-Dihalogen-4-chlor-4,4-difluorbuttersäureestern der Formel

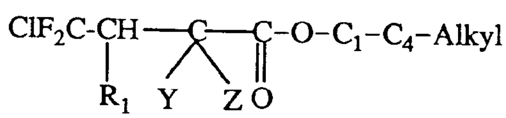

worin $R_1$ die unter Formel I gegebene Bedeutung hat und Y und Z unabhängig voneinander Chlor oder Brom bedeuten, erhalten werden. Letztere α,α-Dihalogenbuttersäureester sind aus der EP-A-2206 bekannt.

Bei geeigneter Wahl des Katalysators und der Reaktionsbedingungen ist es auch möglich, die α-Halogenatome Y und Z stufenweise durch Wasserstoff zu ersetzen.

Die katalytische Dehalogenierungsverfarehn mit Hilfe von Wasserstoff werden in Gegenwart eines Edelmetallkatalysators oder RaneyNickel, gegebenenfalls in Gegenwart eines Halogenwasserstofffängers und eines Lösungsmittels bei einer Temperatur zwischen 0°C und +150°C und bei Normaldruck oder einem Druck bis zu 150 bar durchgeführt.

Die Verbindungen der Formel V können auch durch katalytische Hydrierung mit Wasserstoff aus den 4-Chlor-4,4-difluorcrotonsäurederivaten der Formel VI

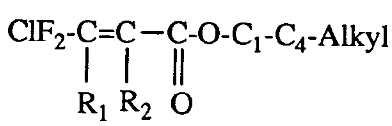

(VI)

worin $R_1$ und $R_2$ die unter Formel I gegebenen Bedeutungen haben, erhalten werden.

Die katalytische Hydrierung der Crotonsäureester der Formel VI wird unter für diesen Reaktionstyp üblichen Bedingungen ausgeführt. So führt man die Reaktion in Gegenwart eines Edelmetallkatalysators oder von Raney-Nickel, vorzugsweise in einem inerten Lösungsmittel, unter einer Wasserstoffatomsphäre bei einem Druck zwischen 1 und 150 bar durch.

Verbindungen der Formel VI, worin $R_1$ und $R_2$ Wasserstoff bedeuten, können auch aus Verbindungen der Formel,

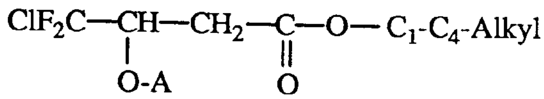

worin A Wasserstoff oder eine Acylgruppe bedeutet, durch β-Elimination nach bekannten Methoden erhalten werden, z.B. Houben Weyl 6/1b 939 (1984). Letztere Verbindungen können aus Verbindungen der Formel

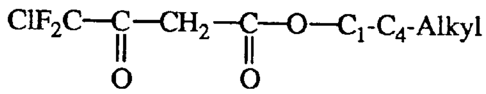

durch Reduktion, z.B. katalytisch mit Wasserstoff nach der von Reuben G. Jones in J. Amer. Chem. Soc., 70 (1948) 144 beschriebenen Methode, hergestellt werden. Die verwendeten β-Ketobuttersäureester sind teilweise bekannt oder können nach bekannten Methoden wie z.B. Claisen Kondensation (Huang Weiynan et al.; Huaxne Xuebao 1983, 41(8) 723; C.A. 100, (1984) 22308s) hergestellt werden. Weiter erhält man diese Verbindungen, indem man Chlordifluoracetylchlorid $ClF_2C\text{-}CO\text{-}Cl$ mit Keten $H_2C{=}C{=}O$ umsetzt und das erhaltene 4-Chlor-4,4-difluoracetessigsäurechlorid der Formel

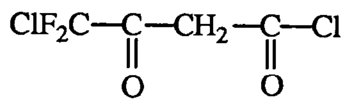

zur freien Säure hydrolysiert und durch Reaktion mit den geeigneten Alkoholen in die Ester überführt Mit Vorteil kann man diese Ester auch direkt durch Umsetzung des Säurechlorids mit einem Alkohol oder Amin erhalten. Beispielsweise ist für Verbindungen der Formel V, worin $R_1$ und $R_2$ Wasserstoff bedeuten, eine Synthese nach folgendem

Schema 1 als vorteilhaft anzusehen:

Schema 1:

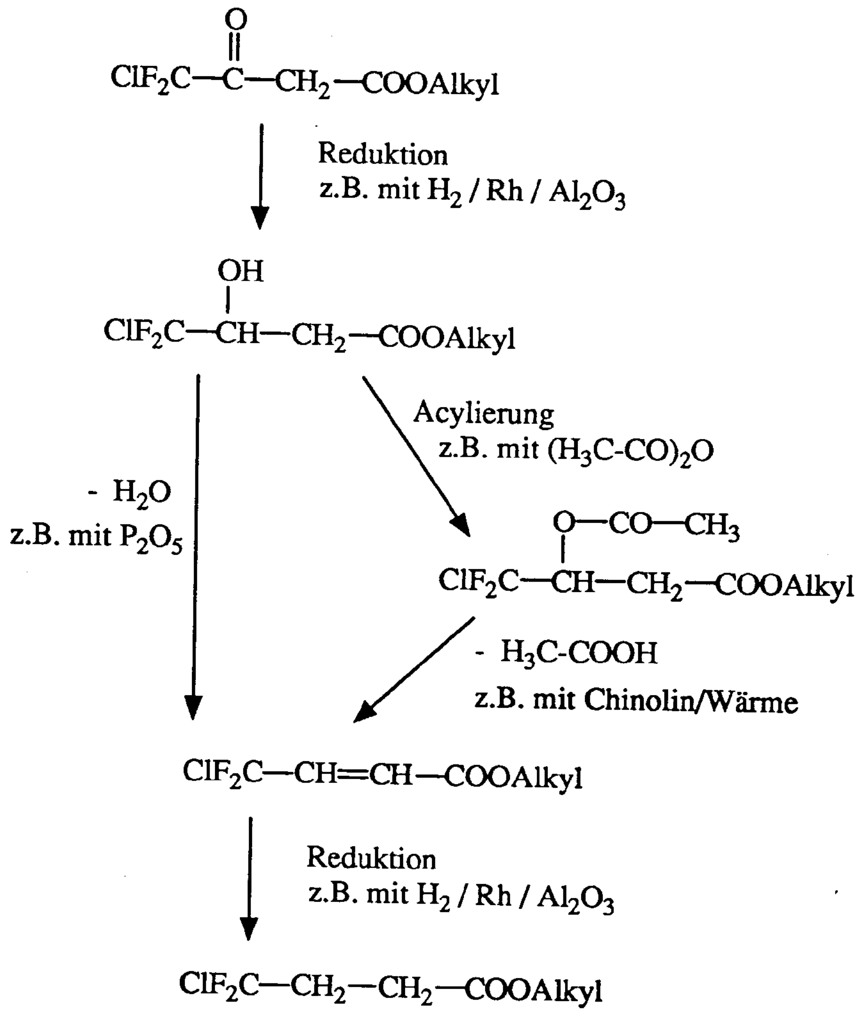


Es wurde nun gefunden, dass die erfindungsgemässen Verbindungen der Formel I bei günstiger Warmblüter-, Fisch- und Pflanzenverträglichkeit wertvolle Wirkstoffe in der Schädlingsbekämpfung sind. Insbesondere betrifft die Anwendung der erfindungsgemässen Wirkstoffe Insekten und Spinnentiere, die in Nutz- und Zierpflanzen in der Landwirtschaft, insbesondere in Baumwoll-, Gemüse- und Obstpflanzungen, im Forst, im Vorrats- und Materialschutz sowie im Hygienesektor insbesondere an Haus- und Nutztieren vorkommen. Sie sind gegen alle oder einzelne Entwicklungsstadien von normal sensiblen aber auch resistenten Arten wirksam. Dabei kann sich ihre Wirkung in unmittelbarer Abtötung der Schädlinge oder erst nach einiger Zeit, beispielsweise bei einer Häutung, oder in einer verminderten Eiablage und/oder Schlupfrate zeigen. Zu den oben erwähnten Schädlingen gehören:
aus der Ordnung Lepidoptera zum Beispiel
Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni und Yponomeuta spp.;
aus der Ordnung Coleoptera zum Beispiel
Agriotes spp., Anthonomus spp., Atomaria linearis, Chaetocnema tibialis, Cosmopolites spp., Curculio spp.,

Dermestes spp., Diabrotica spp., Epilachna spp., Eremnus spp., Leptinotarsa decemlineata, Lissorhoptrus spp. Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.;
aus der Ordnung der Orthoptera zum Beispiel Blatta spp., Blattella spp., Gryllotalpa spp., Leucophaea maderae, Locusta spp., Periplaneta spp. und Schistocerca spp.;
aus der Ordnung der Isoptera zum Beispiel
Reticulitermes spp.; aus der Ordnung der Psocoptera zum Beispiel Liposcelis spp.; aus der Ordnung der Anoplura zum Beispiel Haematopinus spp., Linognathus spp. Pediculus spp., Pemphigus spp. und Phylloxera spp.;
aus der Ordnung der Mallophaga zum Beispiel
Damalinea spp. und Trichodectes spp.;
aus der Ordnung der Thysanoptera zum Beispiel
Frankliniella spp., Hercinothrips spp., Taeniothrips spp., Thrips palmi, Thrips tabaci und Scirtothrips aurantii;
aus der Ordnung der Heteroptera zum Beispiel
Cimex spp., Distantiella theobroma, Dysdercus spp., Euchistus spp. Eurygaster spp. Leptocorisa spp., Nezara spp., Piesma spp., Rhodnius spp., Sahlbergella singularis, Scotinophara spp. und Triatoma spp.;
aus der Ordnung der Homoptera zum Beispiel
Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium corni, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae und Unaspis citri;
aus der Ordnung der Hymenoptera zum Beispiel
Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. und Vespa spp.;
aus der Ordnung der Diptera zum Beispiel
Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp. Oscinella frit, Pegomyia hyoscyami, Phorbia spp., Rhagoletis pomonella, Sciara spp., Stomoxys spp., Tabanus spp., Tannia spp. und Tipula spp.;
aus der Ordnung der Siphonaptera z.B.
Ceratophyllus spp., Xenopsylla cheopis,
aus der Ordnung der Acarina zum Beispiel
Acarus siro, Aceria sheldoni, Aculus schlechtendali, Amblyomma spp., Argas spp., Boophilus spp., Brevipalpus spp., Bryobia praetiosa, Calipitrimerus spp., Chorioptes spp., Dermanyssus gallinae, Eotetranychus carpini, Eriophyes spp., Hyalomma spp., Ixodes spp., Olygonychus pratensis, Omithodoros spp., Panonychus spp., Phyllocoptruta oleivora, Polyphagotarsonemus latus, Psoroptes spp., Rhipicephalus spp., Rhizoglyphus spp., Sarcoptes spp., Tarsonemus spp. und Tetranychus spp.; und
aus der Ordnung der Thysanura zum Beispiel
Lepisma saccharina.

Insbesondere eignen sich die Verbindungen der Formel I in besonderem Masse zur Kontrolle der Schädlinge in Reis- und Baumwollkulturen, wie der Reiszikaden der Familien Delphacidae und Cicadellidae, insbesondere Nilaparvata lugens, Laodelphax striatellus und Nephotettix cincticeps, und der Baumwollschädlinge Heliothis und Spodoptera.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen und der sie enthaltenden Mittel lässt sich durch Zusatz von anderen Insektiziden und/oder Akariziden wesentlich verbreitern und an gegebene Umstände anpassen. Als Zusätze kommen zum Beispiel Vertreter der folgenden Wirkstoffklassen in Betracht: Organische Phosphorverbindungen, Nitrophenole und Derivate, Formamidine, Hamstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und können daher beispielsweise zu Emulsionskonzentraten, direkt versprüh- oder verdünnbaren Lösungen, verdünnten Emulsionen, Spritzpulvern, löslichen Pulvern, Stäubemitteln, Granulaten, auch Verkapselungen in polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, das heisst die den Wirkstoff der Formel I, beziehungsweise Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, zum Beispiel durch inniges Vermischen und/oder Vermahlen der Wirkstoffe mit Streckmitteln, wie beispielsweise mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, beispielsweise für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht sorptive Trägermaterialien Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und-/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie die Natrium- oder Kalium-Salze der Oel- oder Stearinsäure, oder von natürlichen Fettsäuregemischen, die beispielsweise aus Kokosnuss- oder Tallöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, beispielsweise das Natrium- oder Calcium-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind zum Beispiel die Natrium-, Calcium- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie zum Beispiel Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quarternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen

vorzugsweise als Halogenide, Methylsulfate oder Aethylsulfate vor, beispielsweise das Stearyltrimethylammoniumchlorid oder das Benzyl-di-(2-chloräthyl)-äthylammoniumbromid.

Die in der Formulierungstechnik gebräuchlichen Tenside sind beispielsweise in folgenden Publikationen beschrieben:

"1985 International Mc Cutcheon's Emulsifiers & Detergents", Glen Rock NJ USA, 1985",

H. Stache, "Tensid-Taschenbuch", 2. Aufl., C. Hanser Verlag München, Wien 1981,

M. and J. Ash. "Encyclopedia of Surfactants", Vol. I:III, Chemical Publishing Co., New York, 1980-1981.

Die pestiziden Zubereitungen enthalten in der Regel 0,1 bis 99 %, insbesondere 0,1 bis 95 % Wirkstoff der Formel I oder Kombinationen dieses Wirkstoffes mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen. Typische Anwendungskonzentrationen liegen zwischen 0,1 und 1000 ppm, vorzugsweise zwischen 0,1 und 500 ppm. Die Aufwandmengen pro Hektar betragen im allgemeinen 1 bis 1000 g Wirkstoff pro Hektar, vorzugsweise 25 bis 500 g/ha.

Insbesondere setzen sich bevorzugte Formulierungen folgendermassen zusammen: (% = Gewichtsprozent)

Emulgierbare Konzentrate:

| | |
|---|---|
| Aktiver Wirkstoff: | 1 bis 20 %, bevorzugt 5 bis 10 % |
| oberflächenaktives Mittel: | 5 bis 30 %, vorzugsweise 10 bis 20 % |
| flüssiges Trägermittel: | 50 bis 94 %, vorzugsweise 70 bis 85 % |

Stäube:

| | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |

Suspension-Konzentrate:

| | |
|---|---|
| Aktiver Wirkstoff: | 5 bis 75 %, vorzugsweise 10 bis 50 % |
| Wasser: | 94 bis 24 %, vorzugsweise 88 bis 30 % |
| oberflächenaktives Mittel: | 1 bis 40 %, vorzugsweise 2 bis 30 % |

Benetzbare Pülver:

| | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermaterial: | 5 bis 95 %, vorzugsweise 15 bis 90 % |

Granulate:

| | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 % |

Die Mittel können auch weitere Zusätze wie Stabilisatoren, Entschäumer, Konservierungmittel, Viskositätsregulatoren, Bindemittel, Haftmittel sowie Dünger oder andere Wirkstoffe zur Erzielung spezieller Effekte enthalten.

Die folgenden Beispiele dienen der Erläuterung der Erfindung. Sie schränken die Erfindung nicht ein.

Herstellungsbeispiele

Beispiel H1: 4-Chlor-4,4-difluorbuttersäure-äthylester

$$ClF_2C\text{-}CH_2\text{-}CH_2\text{-}COOC_2H_5$$

a) 22,1 g 2,4-Dichlor-4,4-difluorbuttersäure-äthylester werden in 200 ml absolutem Aethanol gelöst. Nach der Zugabe von 8,2 g wasserfreiem Natriumacetat und 2,0 g 5%-igem Platin/Kohle-Katalysator wird bei Normaldruck Wasserstoffgas eingeleitet, bis die Wasserstoffaufnahme 100 % der Theorie beträgt. Nach dem Abfiltrieren des Katalysators und dem Abdestillieren des Aethanols wird das zurückbleibende Oel auf Wasser aufgegossen, die organische Phase abgetrennt, mit Natriumsulfat getrocknet, filtriert und bei Normaldruck rektifiziert. Man erhält 14,0 g 4-Chlor-4,4-difluorbuttersäure-äthylester in Form eines farblosen Oels mit dem Siedepunkt 154-156°C.

Analyse: $C_6H_9ClF_2O_2$ (186,59)
Ber.: C 38,62 % H 4,86 % Cl 19,00 % F 20,36 %
Gef.: C 38,6 % H 4,8 % Cl 19,1 % F 19,9 %

b) 22,1 g 2,4-Dichlor-4,4-difluorbuttersäure-äthylester werden in 100 ml absolutem Tetrahydrofuran gelöst. Nach der Zugabe von 10,7 g 2,6-Dimethylpyridin und 2,0 g 5%-igem Palladium/Kohle-Katalysator wird bei Normaldruck Wasserstoffgas eingeleitet, bis die Wasserstoffaufnahme 100 % der Theorie beträgt. Nach dem Abfiltrieren des Katalysators und dem Abdestillieren des Tetrahydrofurans wird das zwückbleibende Oel auf Wasser aufgegossen, die organische Phase abgetrennt, mit Natriumsulfat getrocknet, filtriert und bei Normaldruck rektifiziert. Man erhält 14,0 g 4-Chlor-4,4-difluorbuttersäure-äthylester in Form eines farblosen Oels mit dem Siedepunkt 154-156°C.

Analyse: $C_6H_9ClF_2O_2$ (186,59)
Ber.: C 38,62 % H 4,86 % Cl 19,00 % F 20,36 %
Gef.: C 38,6 % H 4,8 % Cl 19,1 % F 19,9 %

c) 25,5 g 2,2,4-Trichlor-4,4-difluorbuttersäure-äthylester werden in 200 ml absolutem Aethanol gelöst. Nach der Zugabe von 16,4 g wasserfreiem Natriumacetat und 2,0 g 5%-igem Platin/Kohle-Katalysator wird bei Normaldruck Wasserstoffgas eingeleitet, bis die Wasserstoffaufnahme 2 Aequivalente beträgt. Nach dem Abfiltrieren des Katalysators und dem Abdestillieren des Aethanols wird das zwückbleibende Oel auf Wasser aufgegossen, die organische Phase abgetrennt, mit Natriumsulfat getrocknet, filtriert und bei Normaldruck rektifiziert. Man erhält 14,0 g 4-Chlor-4,4-difluorbuttersäure-äthylester in Form eines farblosen Oels mit dem Siedepunkt 154-156°C.

Analyse: $C_6H_9ClF_2O_2$ (186,59)
Ber.: C 38,62 % H 4,86 % Cl 19,00 % F 20,36 %
Gef.: C 38,6 % H 4,8 % Cl 19,1 % F 19,9 %

Beispiel H2: 4-Chlor-4,4-difluorbuttersäure

$$ClF_2C\text{-}CH_2\text{-}CH_2\text{-}COOH$$

18,6 g 4-Chlor-4,4-difluorbuttersäure-äthylester werden mit 100 ml 2N-NaOH bei Raumtemperatur gerührt bis eine homogene Lösung entstanden ist. Die Lösung wird hierauf auf 150 ml 2N-HCl aufgegossen. Die ausgeschiedene organische Phase wird in Diäthyläther aufgenommen, mit Natriumsulfat getrocknet und nach dem Abdestillieren des Aethers im Vakuum rektifiziert. Man erhält 12,6 g 4-Chlor-4,4-difluorbuttersäure in Form eines farblosen Oels vom Siedepunkt 88-90°C/11 mbar.

Analyse: $C_4H_5ClF_2O_2$ (158,53)
Ber.: C 30,31 % H 3,18 % Cl 22,36 % F 23,97 %
Gef.: C 30,3 % H 3,2 % Cl 22,4 % F 24,2 %

Beispiel H3: 4-Chlor-4,4-difluorbuttersäure-chlorid

$$ClF_2C\text{-}CH_2\text{-}CH_2\text{-}CO\text{-}Cl$$

15,8 g 4-Chlor-4,4-difluorbuttersäure werden mit 50 ml Thionylchlorid und 0,2 ml Dimethylformamid gemischt und innerhalb von 2 Stunden auf +70°C erwärmt und darauf noch 30 Minuten bei +70°C gehalten. Das Rektionsgemisch wird im Vakuum rektifiziert und die beim Siedepunkt von 35-37°C/21 mbar übergehende Flüssigkeit aufgefangen. Man erhält 10,5 g 4-Chlor-4,4-difluorbuttersäurechlorid in Form einer klaren, farblosen

Flüssigkeit.
Analyse: $C_4H_4Cl_2F_2O$ (176,98)
Ber.: C 27,15 % H 2,28 % Cl 40,06 % F 21,47 %
Gef.: C 27,3 % H 2,3 % Cl 40,1 % F 21,4 %

Beispiel H4: 4-Chlor-4,4-difluorbuttersäure

$ClF_2C\text{-}CH_2\text{-}CH_2\text{-}COOH$

15,6 g 4-Chlor-4,4-difluorcrotonsäure werden in 160 ml Tetrahydrofuran gelöst und nach Zugabe von 0,8 g 5% Pd/$BaSO_4$-Katalysator bei Raumtemperatur und Normaldruck mit Wasserstoffgas behandelt, bis die Wasserstoff-Aufnahme 100 % der Theorie beträgt. Nach dem Abfiltrieren des Katalysators und dem Abdestillieren des Lösungsmittels wird das zwückbleibende Oel im Vakuum rektifiziert Man erhält 12 g eines farblosen Oels vom Siedepunkt 88-90°C/11 mbar, das mit der nach Beispiel H2 hergestellten 4-Chlor-4,4-difluorbuttersäure identisch ist.

Beispiel H5: 4-Chlor-4,4-difluor-thiobuttersäure-S-benzylester

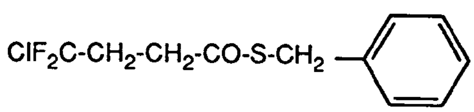

Zu einer Lösung von 4,5 g N,N'-Carbonyldiimidazol in 30 ml Dimethylformamid lässt man bei 0°C innerhalb von 45 Minuten 4,0 g 4-Chlor-4,4-difluorbuttersäure zutropfen. Nachdem die Lösung bei 0°C für 2 Stunden gerührt worden ist, fügt man 2,82 g Benzylmercaptan zu und lässt sich das Gemisch innerhalb von 17 Stunden auf Raumtemperatur erwärmen. Anschliessend fügt man 100 ml Aethylacetat zu und wäscht die organische Lösung mit gesättigter Natriumchloridlösung. Die organische Phase wird über $MgSO_4$ getrocknet und eingedampft. Das erhaltene Rohprodukt wird an Kieselgel chromatographisch gereinigt (Laufmittel: Hexan/Aethylacetat, 9:1). Man erhält so 4,0 g 4-Chlor-4,4-difluor-thiobuttersäure-S-benzylester als Oel, $n_D^{20}$: 1,5220 .

Analog zu den beschriebenen Arbeitsweisen werden auch die in den nachfolgenden Tabelle genannten Verbindungen hergestellt.

Beispiel H6: 4-Chlor-4,4-difluor-thiobuttersäure-S-cyclohexylester

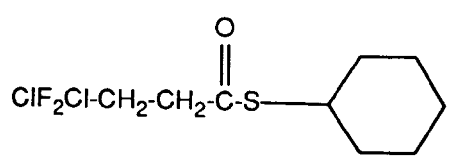

Zu einer Lösung von 2,6 g Cyclohexylmercaptan und 4,5 g Pyridin in 50 ml Toluol lässt man bei 0°C innerhalb von 30 Minuten 4,0 g 4-Chlor-4,4-difluor-buttersäurechlorid zutropfen. Nach 16 Stunden Rühren bei RT wird das Reaktionsgemisch mit 150 ml Diäthyläther verdünnt und anschliessend mit je 50 ml IN HCl, gesättigter $NaHCO_3$- und gesättigter NaCl-Lösung gewaschen. Die organische Phase wird dann über $MgSO_4$ getrocknet, filtriert und eingdampft. Das erhaltene Rohprodukt wird bei +90°C/5 mbar destilliert. Man erhält 3,8 g 4-Chlor-4,4-difluor-thiobuttersäure-S-cyclohexylester als Oel, $n_D^{23}$ : 1,4748.

Tabelle 1:

$ClF_2C-CH_2-CH_2-CO-S-R_3$

| Verb. Nr. | $R_3$ | physikalische Daten |
|---|---|---|
| 1.01 | $-CH_3$ | |
| 1.02 | $-C_2H_5$ | |
| 1.03 | $-C_3H_7$-n | $n_D^{20}$ : 1,4489 |
| 1.04 | $-C_4H_9$-n | $n_D^{20}$ : 1,4492 |
| 1.05 | $-C_5H_{11}$-n | |
| 1.06 | $-C_6H_{13}$-n | $n_D^{23}$ : 1,4502 |
| 1.07 | $-C_9H_{19}$-n | |
| 1.08 | $-C_{12}H_{25}$-n | $n_D^{23}$ : 1,4563 |
| 1.09 | $-C_{16}H_{33}$-n | |
| 1.10 | $-C_{14}H_{29}$-n | |
| 1.11 | $-C_{18}H_{37}$-n | |
| 1.12 | $-C(CH_3)_3$ | |
| 1.13 | $-C(CH_3)_2CH_2-CH_3$ | |
| 1.14 | $-CH(CH_3)_2$ | |
| 1.15 | $-CH(CH_3)-CH_2-CH_3$ | |
| 1.16 | $-CH_2-CH(CH_3)_2$ | |
| 1.17 | $-CH_2-CH(CH_3)-CH_2-CH_3$ | |
| 1.18 | $-CH_2-CH_2-CH(CH_3)_2$ | |
| 1.19 | $-CH_2COO-CH_3$ | |
| 1.20 | $-CH_2-COO-C_2H_5$ | $n_D^{23}$ : 1,4551 |
| 1.21 | $-CH_2-COOCH_2-CH(C_2H_5)-(CH_2)_3CH_3$ | $n_D^{23}$ : 1,4559 |
| 1.22 | $-CH_2-CO-NH-C_6H_5$ | |
| 1.23 | $-CH_2-CO-NHCH_3$ | |
| 1.24 | $-C(CH_3)_2-CH_2-C(CH_3)_3$ | |
| 1.25 | $-CH_2-CH_2-C_6H_5$ | |
| 1.26 | $-CH_2-CH_2-CH_2-C_6H_5$ | |

Tabelle 1: (Fortsetzung)

| Verb. Nr. | $R_3$ | physikalische Daten |
|---|---|---|
| 1.27 | 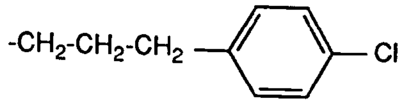 | |
| 1.28 | $-CH_2-CH_2-COO-CH_3$ | |
| 1.29 | $-CH_2-CH_2-CH_2-Cl$ | |
| 1.30 | $-CH_2-CH_2-N(CH_3)_2$ | |
| 1.31 | $-CH_2-CH_2-O-COCH_3$ | |
| 1.32 | $-CH_2-CH_2-O-CH_2CH_3$ | |
| 1.33 | $-CH_2-CH_2-S-CH_2-CH_2-CH_3$ | |
| 1.34 | 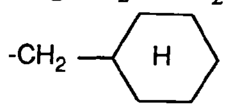 | |
| 1.35 | 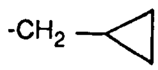 | |
| 1.36 | $-CH_2-C_6H_5$ | $n_D^{20}$ : 1,5220 |
| 1.37 | $-CH_2-C_6H_4-Cl(2)$ | |
| 1.38 | $-CH_2-C_6H_4-Cl(4)$ | |
| 1.39 | $-CH_2-C_6H_4-CH_3(4)$ | |
| 1.40 | $-CH_2-C_6H_4-CH_3(3)$ | |
| 1.41 | $-CH_2-C_6H_4-CH_3(2)$ | |
| 1.42 | $-CH_2-C_6H_4-OCH_3(4)$ | |
| 1.43 | $-CH_2-C_6H_4-NO_2(4)$ | |
| 1.44 | $-CH_2-C_6H_4-NO_2(3)$ | |
| 1.45 | $-CH_2-C_6H_4-F(4)$ | |
| 1.46 | $-CH_2-C_6H_4-CF_3(3)$ | |
| 1.47 | $-CH_2-C_6H_4-OCF_3(4)$ | |
| 1.48 | 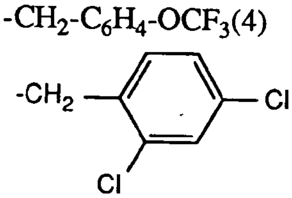 | |
| 1.49 | 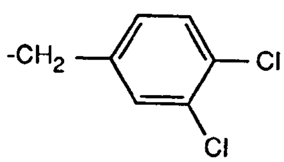 | |

Tabelle 1: (Fortsetzung)

| Verb. Nr. | $R_3$ | physikalische Daten |
|---|---|---|
| 1.50 | 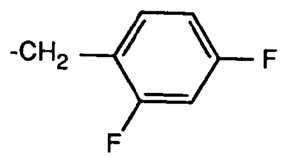 | |
| 1.51 | 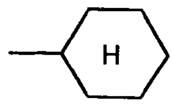 | $n_D^{20}$ : 1,4748 |
| 1.52 | 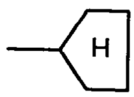 | |
| 1.53 | $-CH_2-CH=CH-CH_3$ | |
| 1.54 | $-C_6H_5$ | $n_D^{20}$ : 1,5249 |
| 1.55 | $-C_6H_4-Cl(2)$ | |
| 1.56 | $-C_6H_4-Cl(3)$ | $n_D^{20}$ : 1,5379 |
| 1.57 | $-C_6H_4-Cl(4)$ | |
| 1.58 | $-C_6H_4-F(4)$ | |
| 1.59 | $-C_6H_4-Br(4)$ | $n_D^{23}$ : 1,5531 |
| 1.60 | $-C_6H_4-CH_3(4)$ | $n_D^{20}$ : 1,5233 |
| 1.61 | $-C_6H_4-OCH_3(4)$ | |
| 1.62 | $-C_6H_4-NHCOCH_3(4)$ | |
| 1.63 | $-C_6H_4-C(CH_3)_3(4)$ | |
| 1.64 | $-C_6H_4-OCH_3$ | |
| 1.65 | $-C_6H_4-CH_3(3)$ | |
| 1.66 | $-C_6H_4-CH_3(2)$ | |
| 1.67 | $-C_6H_4-Br(3)$ | |
| 1.68 | $-C_6H_4-C_2H_5(2)$ | |
| 1.69 | $-C_6H_4-Br(2)$ | $n_D^{23}$ : 1,5509 |
| 1.70 | $-C_6H_4-OCF_3(4)$ | |
| 1.71 | $-C_6H_4-CF_3(4)$ | |
| 1.72 | $-C_6H_4-CF_3(3)$ | $n_D^{23}$ : 1,4788 |
| 1.73 | 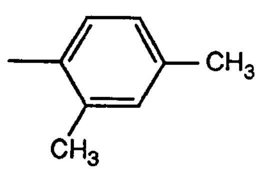 | $n_D^{22}$ : 1518 |

Tabelle 1: (Fortsetzung)

| Verb. Nr. | $R_3$ | physikalische Daten |
|---|---|---|
| 1.74 | 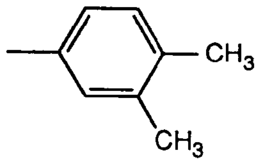 | $n_D^{23}$ : 1,5247 |
| 1.75 | 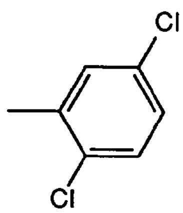 | |
| 1.76 | 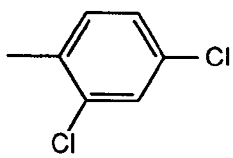 | |
| 1.77 | 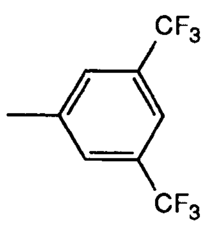 | |
| 1.78 | 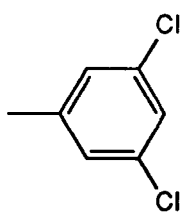 | |
| 1.79 | 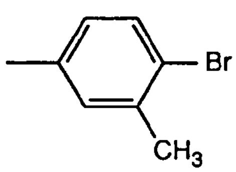 | |
| 1.80 | 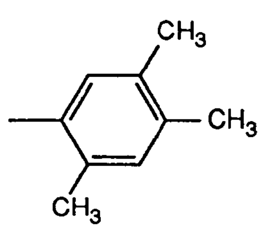 | |

Tabelle 1: (Fortsetzung)

| Verb. Nr. | $R_3$ | physikalische Daten |
|---|---|---|
| 1.81 | 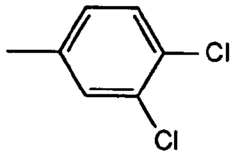 | $n_D^{23}$ : 1,5523 |
| 1.82 | 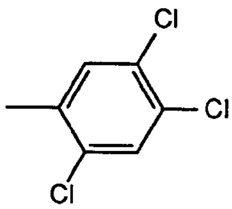 | Smp. 39-41°C |
| 1.83 | 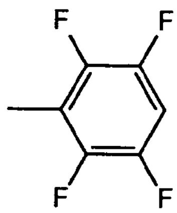 | |
| 1.84 | 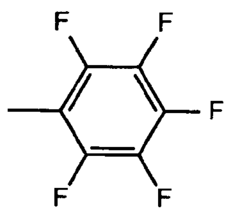 | |
| 1.85 | 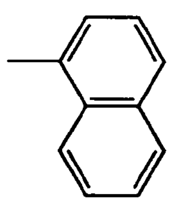 | |
| 1.86 | 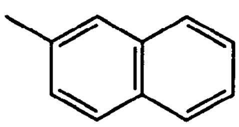 | Smp. 58-60°C |
| 1.87 | 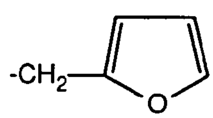 | $n_D^{20}$ : 1,4930 |
| 1.88 | 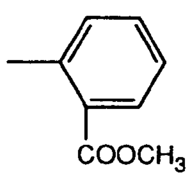 | $n_D^{23}$ : 1,5297 |

Tabelle 1: (Fortsetzung)

| Verb. Nr. | $R_3$ | physikalische Daten |
|---|---|---|
| 1.89 | $-CH_2-CO-O-(CH_2)_2-OCH_3$ | $n_D^{23}$ : 1,4600 |
| 1.90 | 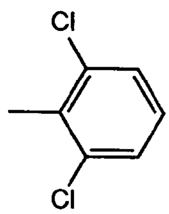 | $n_D^{23}$ : 1,5222 |
| 1.91 | $-CH_2-CO-O-C_{10}H_{21}-n$ | $n_D^{23}$ : 1,4576 |
| 1.92 | $-CH_2-CO-O-C_{18}H_{37}-n$ | Smp. 46-48°C |
| 1.93 | $-CH_2-CO-O-$ 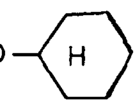 | $n_D^{23}$ : 1,4770 |
| 1.94 | $-CH_2-CO-O-C_{12}H_{25}-n$ | $n_D^{24}$ : 1,4580 |

Formulierungsbeispiele (% = Gewichtsprozent)

| Beispiel F1: Emulsions-Konzentrate | a) | b) | c) |
|---|---|---|---|
| Wirkstoff Nr. 1.36 | 25 % | 40 % | 50 % |
| Ca-Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| Beispiel F2: Lösungen | a) | b) | c) | d) |
|---|---|---|---|---|
| Wirkstoff Nr. 1.54 oder 1.56 | 80 % | 10 % | 5 % | 95 % |
| Aethylenglykolmonomethyläther | 20 % | - | - | - |

| | | | | |
|---|---|---|---|---|
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| Beispiel F3: Granulate | a) | b) |
|---|---|---|
| Wirkstoff Nr. 1.54 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| Beispiel F4: Stäubemittel | a) | b) |
|---|---|---|
| Wirkstoff Nr. 1.60 oder 1.36 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

| Beispiel F5: Spritzpulver | a) | b) | c) |
|---|---|---|---|
| Wirkstoff aus Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalin-sulfonat | - | 6 % | 10 % |
| Octylphenolpolyäthylenglykol- | | | |

| | | | |
|---|---|---|---|
| äther (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle gut vermahlen. Man erhält Spritzpulver, die sich mit Wasser zu Suspensionen jeder gewünschten Konzentration verdünnen lassen.

Beispiel F6: Emulsions-Konzentrat

| | |
|---|---|
| Wirkstoff aus Tabelle 1 | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Beispiel F7: Stäubemittel | a) | b) |
|---|---|---|
| Wirkstoff aus Tabelle 1 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

Beispiel F8: Extruder-Granulat

| | |
|---|---|
| Wirkstoff aus Tabelle 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

Beispiel F9: Umhüllungs-Granulat

| | |
|---|---|
| Wirkstoff aus Tabelle 1 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

Beispiel F10: Suspensions-Konzentrat

| | |
|---|---|
| Wirkstoff aus Tabelle 1 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%-ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Beispiel B1: Wirkung gegen Boophilus microplus

Vollgesogene adulte Zecken Weibchen werben auf eine PVC Platte aufgeklebt und mit einem Wattebausch überdeckt. Zur Behandlung werden die Testtiere mit 10 ml einer wässrigen Testlösung, die 125 ppm des zu prüfenden Wirkstoffes enthält übergossen. Anschliessend wird der Wattebausch entfernt und die Zecken werden für 4 Wochen zur Eiablage inkubiert. Die Wirkung gegen Boophilus microplus zeigt sich entweder beim Weibchen als Mortalität oder Sterilität, oder bei den Eiern als ovizide Wirkung.

Verbindungen gemäss Tabelle 1 zeigen in diesem Test gute Wirkung gegen Boophilus microplus. Insbesondere die Verbindungen 1.03, 1.04, 1.36, 1.51, 1.54, 1.56, 1.60, 1.72, 1.73, 1.81, 1.82, 1.86, 1.87 und 1.88 zeigen eine Wirkung über 80 %.

Beispiel B2: Ovizide Wirkung auf Heliothis virescens

Auf Filterpapier abgelegte Eier von Heliothis virescens werden für kurze Zeit in eine acetonisch-wässrige Testlösung, die 400 ppm des zu prüfenden Wirkstoffes enthält, eingetaucht. Nach dem Antrocknen der Testlösung werden die Eier in Petrischalen inkubiert. Nach 6 Tagen wird der prozentuale Schlupf der Eier im Vergleich zu unbehandelten Kontrollansätzen ausgewertet (% Schlupfreduktion).

Verbindungen gemäss Tabelle 1 zeigen in diesem Test gute Wirkung gegen Heliothis virescens.

Beispiel B3: Wirkung gegen Aonidiella aurantii

Kartoffelknollen werden mit Wanderlarven ("Crawlern") von Aonidiella aurantii (rote Citrus-Schildlaus) besiedelt. Nach etwa 2 Wochen werden die Kartoffeln in eine wässrige. Emulsions- resp. Suspensions-Spritzbrühe getaucht, die den zu prüfenden Wirkstoff in einer Konzentration von 400 ppm enthält. Nach dem Abtrocknen der so behandelten Kartoffelknollen werden diese in einem Plastikbehälter inkubiert. Zur Auswertung wird 10- 12 Wochen später die Ueberlebensrate der Wanderlarven der ersten Folgegeneration der behandelten Schildlaus-Population mit derjenigen der unbehandelten Kontrollansätze verglichen.

Verbindungen gemäss Tabelle 1 zeigen in diesem Test gute Wirkung gegen Aonidiella aurantii. Insbesondere die Verbindungen 1.36, 1.54, 1.56 und 1.60 zeigen eine Wirkung über 80 %.

Beispiel B4: Wirkung gegen Nilaparvata lugens

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.

Verbindungen der Tabelle 1 zeigen eine gute Wirkung gegen Nilaparvata lugens in diesem Test. Insbesondere die Verbindungen 1.04, 1.36, 1.51, 1.54, 1.56, 1.60 und 1.87 zeigen eine Wirkung über 80 %.

Beispiel B5: Wirkung gegen Tetranychus urticae

Junge Bohnenpflanzen werden mit einer Mischpopulation von Tetranychus urticae besiedelt und 1 Tag später mit einer wässrigen Emulsions-Spritzbrühe, die 400 ppm des Wirkstoffes enthält, besprüht. Die Pflanzen werden anschliessend für 6 Tage bei 25°C inkubiert und danach ausgewertet. Aus dem Vergleich der Anzahl toter Eier, Larven und Adulten auf den behandelten zu denjenigen auf den unbehandelten Pflanzen wird die prozentuale Reduktion der Population (% Wirkung) bestimmt.

Verbindungen der Tabelle 1 zeigen eine gute Wirkung gegen Tetranychus urticae in diesem Test. Insbesondere die Verbindungen 1.36, 1.60, 1.74, 1.81, 1.82 und 1.86 zeigen eine Wirkung über 80 %.

Beispiel B6: Ovizide Wirkung auf Lobesia botrana

Auf Filterpapier abgelegte Eier von Lobesia botrana werden für kurze Zeit in eine acetonisch-wässrige Testlösung, die 400 ppm des zu prüfenden Wirkstoffes enthält, eingetaucht. Nach dem Antrocknen der Testlösung werden die Eier in Petrischalen inkubiert. Nach 6 Tagen wird der prozentuale Schlupf der Eier im Vergleich zu unbehandelten Kontrollansätzen ausgewertet (% der Schlupfreduktion).

Verbindungen gemäss Tabelle 1 zeigen in diesem Test gute Wirkung gegen Lobesia botrana.

Beispiel B7: Wirkung gegen Aphis craccivora

Erbsenkeimlinge werden mit Aphis craccivora infiziert und anschliessend mit einer Spritzbrühe, die 400 ppm des Wirkstoffes enthält, besprüht und bei 20°C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (% Wirkung) bestimmt.

Verbindungen der Tabelle 1 zeigen eine gute Wirkung gegen Aphis craccivora in diesem Test. Insbesondere die Verbindungen 1.03, 1.06, 1.36, 1.51, 1.54, 1.56, 1.59, 1.60, 1.69, 1.73, 1,74, 1.81, 1.82, 1.86, 1.87, 1.88 und 1.89 zeigen eine Wirkung über 80 %.

Beispiel B8: Systemische Wirkung gegen Myzus persicae

Erbsenkeimlinge werden mit Myzus persicae infiziert, anschliessend mit den Wurzeln in eine Spritzbrühe, die 400 ppm des Wirkstoffes enthält, gestellt und bei 20°C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (% Wirkung) bestimmt.

Verbindungen der Tabelle 1 zeigen eine gute Wirkung gegen Myzus persicae in diesem Test. Insbesondere die Verbindungen 1.51, 1.56, 1.60 und 1.87 zeigen eine Wirkung über 80 %.

Beispiel B9: Systemische Wirkung gegen Nilaparvata lugens

Töpfe mit Reispflanzen werden in eine wässrige EmulsionsLösung, die 400 ppm des Wirkstoffes enthält, gestellt. Anschliessend werden die Reispflanzen mit I.arven des 2. und 3. Stadiums besiedelt. 6 Tage später erfolgt die Auswertung. Aus dem Vergleich der Anzahl Zikaden auf den behandelten zu denjenigen auf den unbehandelten Pflanzen wird die prozentuale Reduktion der Population (% Wirkung) bestimmt.

Verbindungen der Tabelle 1 zeigen eine gute Wirkung gegen Nilaparvata lugens in diesem Test. Insbesondere die Verbindungen 1.03, 1.04, 1.06, 1.08, 1.21, 1.36, 1.51, 1.54, 1.56, 1.59, 1.60, 1.69, 1.72, 1.73, 1.74, 1.81, 1.82, 1.86, 1.87, 1.88, 1.89 und 1.90 zeigen eine Wirkung über 80 %.

Beispiel B10: Ovizide Wirkung von Adoxophyes reticulana

Auf Filterpapier abgelegte Eier von Adoxophyes reticulana werden für kurze Zeit in eine acetonisch-wässrige Testlösung, die 400 ppm des zu prüfenden Wirkstoffes enthält, eingetaucht. Nach dem Antrocknen der Testlösung werden die Eier in Petrischalen inkubiert. Nach 6 Tagen wird der prozentuale Schlupf der Eier im Vergleich zu unbehandelten Kontrollansätzen ausgewertet (% Schlupfreduktion).

Verbindungen gemäss Tabelle 1 zeigen in diesem Test gute Wirkung gegen Adoxophyes reticulana. Insbesondere die Verbindungen 1.03, 1.04, 1.36, 1.54 und 1.60 zeigen eine Wirkung über 80 %.

Beispiel B11: Ovo/larvizide Wirkung auf Heliothis virescens

Auf Baumwolle abgelegte Eier von Heliothis virescens werden mit einer wässrigen Emulsions-Spritzbrühe, die 400 ppm des Wirkstoffes enthält, besprüht. Nach 8 Tagen wird der prozentuale Schlupf der Eier und die Ueberlebensraten der Raupen im Vergleich zu unbehandelten Kontrollansätzen ausgewertet (% Reduktion der Population).

Verbindungen gemäss Tabelle 1 zeigen in diesem Test gute Wirkung gegen Heliothis virescens. Insbesondere die Verbindungen 1.03, 1.04, 1.06, 1.08, 1.21, 1.36, 1.51, 1.54, 1.56, 1.59, 1.60, 1.69, 1.72, 1.73, 1.74, 1.81, 1.82, 1.86, 1.87, 1.88, 1.89 und 1.90 zeigen eine Wirkung über 80 %.

Beispiel B12: Wirkung gegen Dermanyssus gallinae

In einem nach oben offenen Glasbehälter werden 2 bis 3 ml einer 100 ppm Wirkstoff enthaltenden Lösung und ca. 200 Milben in unterschiedlichen Entwicklungsstadien gegeben. Anschliessend wird der Behälter mit einem Wattebausch verschlossen, 10 Minuten lang bis zur vollständigen Benetzung der Milben geschüttelt und dann kurzfristig umgekehrt, damit die restliche Testlösung von der Watte aufgenommen werden kann. Nach 3 Tagen wird die Mortalität der Milben ermittelt.

Verbindungen gemäss Tabelle 1 zeigen gute Wirkung gegen Dermanyssus gallinae in diesem Test. Insbesondere die Verbindungen 1.03, 1.04, 1.36, 1.51, 1.54, 1.56 und 1.60 zeigen eine Wirkung über 80 %.

Beispiel B13: Wirkung gegen Heliothis virescens Raupen

Junge Sojapflanzen werden mit einer wässrigen Emulsions-Spritzbrühe, die 400 ppm des Wirkstoffes enthält, besprüht. Nach dem Antrocknen des Spritzbelages werden die Sojapflanzen mit 10 Raupen des ersten Stadiums von Heliothis virescens besiedelt und in einen Plastikbehälter gegeben. 6 Tage später erfolgt die Auswertung. Aus dem Vergleich der Anzahl toter Raupen und des Frasschadens auf dem behandelten zu denjenigen auf den unbehandelten Pflanzen wird die prozentuale Reduktion der Population bzw. die prozentuale Reduktion des Frassschadens (% Wirkung) bestimmt.

Verbindungen gemäss Tabelle 1 zeigen eine gute Wirkung gegen Heliothis virescens in diesem Test. Insbesondere die Verbindungen 1.36, 1.54, 1.56 und 1.60 zeigen eine Wirkung über 80 %.

Beispiel B14: Ovizide Wirkung auf Cydia pomonella

Auf Filterpapier abgelegte Eier von Cydia pomonella werden für eine kurze Zeit in eine acetonisch-wässrige Testlösung, die 400 ppm des zu prüfenden Wirkstoffes enthält, eingetaucht. Nach dem Antrocknen der Testlösung werden die Eier in Petrischalen inkubiert. Nach 6 Tagen wird der prozentuale Schlupf der Eier im Vergleich zu unbehandelten Kontrollansätzen ausgewertet (% Schlupfreduktion).

Verbindungen gemäss Tabelle 1 zeigen in diesem Test gute Wirkung gegen Cydia pomonella. Insbesondere die Verbindungen 1.03 und 1.04 zeigen eine Wirkung über 80 %.

Beispiel B15: Wirkung gegen Diabrotica balteata Larven

Maiskeimlinge werden mit einer wässrigen Emulsions-Spritzbrühe, die 400 ppm des Wirkstoffes enthält, besprüht. Nach dem Antrocknen des Spritzbelages werden die Maiskeimlinge mit 10 Larven des zweiten Stadiums von Diabrotica balteata besiedelt und in einen Plastikbehälter gegeben. 6 Tage später erfolgt die Auswertung. Aus dem Vergleich der Anzahl toter Larven auf den behandelten zu denjenigen auf den unbehandelten Pflanzen wird die prozentuale Reduktion der Population (% Wirkung) bestimmt.

Verbindungen der Tabelle 1 zeigen eine gute Wirkung gegen Diabrotica balteata in diesem Test. Insbesondere die Verbindungen 1.60 und 1.81 zeigen eine Wirkung über 80 %.

Beispiel B16: Wirkung gegen Bemisia tabaci

Buschbohnen-Pflanzen werden in Gazekäfige gestellt und mit Adulten von Bemisia tabaci (Weisse Fliegen) besiedelt. Nach erfolgter Eiablage werden alle Adulten entfernt und 10 Tage später die Pflanzen mit den darauf befindenden Nymphen mit einer wässrigen Emulsions-Spritzbrühe der zu prüfenden Wirkstoffe (Konzentration 400 ppm) behandelt. Die Auswertung erfolgt 14 Tage nach der Wirkstoff-Applikation auf %-Schlupf im Vergleich zu den unbehandelten Kontrollansätzen.

Verbindungen gemäss Tabelle 1 zeigen in diesem Test gute Wirkung gegen Bemisia tabaci. Insbesondere die Verbindung 1.88 zeigt eine Wirkung über 80 %.

Beispiel B17: Wirkung gegen Diabrotica balteata Eier

20 bis 50 auf Tuchfilter abgelegte Eier von Diabrotica balteata werden in eine Petrischale gegeben und mit einer wässrigen Emulsions-Spritzbrühe, die 400 ppm des Wirkstoffes enthält, behandelt. Die Petrischalen werden bei 24°C inkubiert. Nach 7 Tagen wird der prozentuale Schlupf der Eier im Vergleich zu unbehandelten Kontrollansätzen ausgewertet (% Schlupfreduktion).

Verbindungen der Tabelle 1 zeigen eine gute Wirkung gegen Diabrotica balteata in diesem Test.

Beispiel B18: Wirkung gegen Bemisia tabaci Eier

Buschbohnen-Pflanzen werden in Gazekäfige gestellt und mit Adulten von Bemisia tabaci (Weisse Fliege) besiedelt. Nach erfolgter Eiablage werden alle Adulten entfernt und 2 Tage später die Pflanzen mit den darauf befindenden Nymphen mit einer wässrigen Emulsions-Spritzbrühe der zu prüfenden Wirkstoffe (Konzentration 400 ppm) behandelt. Die Auswertung erfolgt 10 Tage nach der Wirkstoff-Applikation auf %-Schlupf im Vergleich zu den unbehandelten Kontrollansätzen.

Verbindungen gemäss Tabelle 1 zeigen in diesem Test gute Wirkung gegen Bemisia tabaci.

Beispiel B19: Wirkung gegen Crocidolomia binotalis Raupen

Junge Kohlpflanzen werden mit einer wässrigen Emulsions-Spritzbrühe, die 400 ppm des Wirkstoffes enthält, besprüht. Nach dem Antrocknen des Spritzbelages werden die Kohlpflanzen mit 10 Raupen des dritten Stadiums von Crocidolomia binotalis besiedelt und in einen Plastikbehälter gegeben. 3 Tage später erfolgt die Auswertung. Aus dem Vergleich der Anzahl toter Raupen und des Frasschadens auf den behandelten zu denjenigen auf den unbehandelten Pflanzen wird die prozentuale Reduktion des Frasschadens (% Wirkung) bestimmt.

Verbindungen der Tabelle 1 zeigen eine gute Wirkung gegen Crocidolomia binotalis in diesem Test. Insbesondere die Verbindung 1.36 zeigt eine Wirkung über 80 %.

Beispiel B20: Wirkung gegen Anthonomus grandis Adulte

Junge Baumwollpflanzen werden mit einer wässrigen Emulsions-Spritzbrühe, die 400 ppm des Wirkstoffes enthält, besprüht. Nach dem Antrocknen des Spritzbelages werden die Baumwollpflanzen mit 10 Adultenvon Anthonomus grandis besiedelt und in einen Plastikbehälter gegeben. 3 Tage später erfolgt die Auswertung. Aus dem Vergleich der Anzahl toter Käfer und des Frasschadens auf den behandelten zu denjenigen auf den unbehandelten Pflanzen wird die prozentuale Reduktion der Population bzw. die prozentuale Reduktion des Frasschadens (% Wirkung) bestimmt.

Verbindungen der Tabelle 1 zeigen eine gute Wirkung gegen Anthonomus grandis in diesem Test. Insbesondere die Verbindungen 1.03, 1.06, 1.08, 1.21, 1.59, 1.69, 1.72, 1.73, 1.74, 1.81, 1.82, 1.86, 1.87, 1.88 und 1.90 zeigen eine Wirkung über 80 %.

Beispiel B21: Systemische Wirkung gegen Nephotettix cincticeps

Töpfe mit Reispflanzen werden in eine wässrige Emulsions-Lösung, die 400 ppm des Wirkstoffes enthält, gestellt. Anschliessend werden die Reispflanzen mit l.arven des 2. und 3. Stadiums besiedelt. 6 Tage später erfolgt die Auswertung. Aus dem Vergleich der Anzahl Zikaden auf den behandelten zu denjenigen auf den unbehandelten Pflanzen wird die prozentuale Reduktion der Population (% Wirkung) bestimmt.

Verbindungen der Tabelle 1 zeigen eine gute Wirkung gegen Nephotettix cincticeps in diesem Test.

Beispiel 22: Wirkung gegen Ctenocephalides felis

20 bis 25 Floheier werden in eine waagrecht stehende 50-ml-Zellkulturflasche gegeben, in der zuvor 15 g Flohlarven-Nährmedium, welches 100 ppm des zu prüfenden Wirkstoffes enthält, vorgelegt wurde. Die Testflaschen werden in einem Brutschrank bei 26-27°C und 60-70 % Luftfeuchtigkeit bebrütet. Nach 21 Tagen wird die Anwesenheit von adulten Flöhen, nicht geschlüpften Puppen und Larven überprüft.

Verbindungen der Tabelle 1 zeigen eine gute Wirkung gegen Ctenocephalides felis in diesem Test. Insbesondere die Verbindungen 1.36 und 1.56 zeigen eine Wirkung über 80 %.

Beispiel 23: Frasswirkung gegen Ctenocephalides felis (systematisch)

20 Adulte Flöhe der Art Ctenocephalides felis werden in einen flachen runden Käfig gegeben, der auf beiden Seiten mit Gaze verschlossen ist. Auf diesen Käfig wird nun ein Gefäss gestellt, das auf der unteren Seite mit einer Parafilmmembran verschlossen ist. Das Gefäss enthält Blut, das 50 ppm des Wirkstoffes enthält und konstant auf 37°C erwärmt wird. Die Flöhe nehmen das Blut durch die Membran auf. 24 und 48 Stunden nach Ansatz erfolgt die Auswertung. Aus dem Vergleich der Anzahl toter Flöhe mit behandeltem Blut zu denjenigen mit unbehandeltem Blut wird die prozentuale Reduktion der Population (% Wirkung) bestimmt. 24 Stunden nach Behandlung wird das Blut durch neues ebenfalls behandeltes ersetzt.

Verbindungen der Tabelle 1 zeigen eine gute Wirkung gegen Ctenocephalides felis in diesem Test.

## Patentansprüche

1. 4-Chlor-4,4-difluorthiobuttersäure-Derivate Formel I

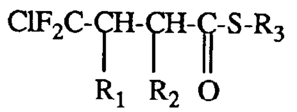

(I)

worin
$R_1$ und $R_2$ unabhängig voneinander Wasserstoff, $C_1$-$C_4$-Alkyl oder $C_1$-$C_4$-Halogenalkyl und
$R_3$ Wasserstoff oder einen organischen Rest bedeuten.

2. Verbindungen gemäss Anspruch 1, dadurch gekennzeichnet, dass $R_3$ für Wasserstoff oder für jeweils substituiertes oder unsubstituiertes $C_1$-$C_{20}$-Alkyl, $C_3$-$C_6$-Cycloalkyl, $C_3$-$C_{10}$-Alkenyl, Benzyl oder Aryl steht.

3. Verbindungen gemäss Anspruch 1, dadurch gekennzeichnet, dass $R_3$ Wasserstoff, $C_1$-$C_{20}$-Alkyl, $C_3$-$C_6$-Cycloalkyl, $C_3$-$C_{10}$-Alkenyl, Aryl, $C_3$-$C_{10}$-Halogenalkenyl; durch Halogen substituiertes $C_3$-$C_6$-Cycloalkyl; durch Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_6$-Alkoxycarbonyl, $C_1$-$C_4$-Alkylthio oder Nitro substituiertes Aryl; oder durch Halogen, Di-$C_1$-$C_4$-alkylamino, $C_1$-$C_4$-Alkoxy, $C_1$-$C_4$-Alkylthio, $C_1$-$C_{20}$-Alkoxycarbonyl, $C_3$-$C_7$Cycloalkoxycarbonyl, $C_1$-$C_4$-Alkylcarbonyloxy, $C_3$-$C_6$-Cycloalkyl, Aryl, $C_1$-$C_{10}$-Alkylaminocarbonyl oder Anilinocarbonyl substituiertes $C_1$-$C_{20}$-Alkyl steht; wobei die Arylgruppen jeweils durch Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder Nitro substituiert sein können.

4. Verbindungen gemäss Anspruch 1, dadurch gekennzeichnet, dass $R_1$ und $R_2$ unabhängig voneinander Wasserstoff bedeuten.

5. Verbindungen gemäss Anspruch 3, dadurch gekennzeichnet, dass $R_3$ für $C_1$-$C_{20}$-Alkyl, $C_3$-$C_6$-Cycloalkyl, $C_3$-$C_{10}$-Alkenyl, Phenyl, Naphthyl, $C_3$-$C_{10}$-Halogenalkenyl, durch Fluor, Chlor oder Brom substituiertes $C_3$-$C_6$-Cycloalkyl, durch Fluor, Chlor, Brom, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_4$-Alkoxycarbonyl, $C_1$-$C_3$-Alkylthio oder Nitro substituiertes Phenyl, oder durch Fluor, Chlor, Brom, Di-$C_1$-$C_4$-alkylamino, $C_1$-$C_4$-Alkoxy, $C_1$-$C_4$-Alkylthio, $C_1$-$C_{10}$-Alkylcarbonyl, $C_1$-$C_{20}$-Alkoxycarbonyl, $C_3$-$C_7$-Cycloalkoxycarbonyl, $C_1$-$C_4$-Alkylcarbonyloxy, $C_3$-$C_6$-Cycloalkyl, Phenyl, $C_1$-$C_{10}$-Alkylaminocarbonyl oder Anilinocarbonyl substituiertes $C_1$-$C_{20}$-Alkyl steht, wobei die Phenylgruppen jeweils durch Fluor, Chlor, Brom, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio oder Nitro substituiert sein können.

6. Verbindungen gemäss Anspruch 1, dadurch gekennzeichnet, dass $R_1$ und $R_2$ unabhängig voneinander Wasserstoff, $R_3$ Phenyl, Benzyl, oder jeweils durch Fluor, Chlor, Brom, $C_1$-$C_3$-Alkyl, $C_1$-$C_3$-Halogenalkyl, $C_1$-$C_3$-Alkoxy, $C_1$-$C_4$-Alkoxycarbonyl, $C_1$-$C_3$-Halogenalkoxy, $C_1$-$C_3$-Alkylthio oder Nitro, substituiertes Phenyl oder Benzyl bedeuten.

7. Verbindungen gemäss Anspruch 1, dadurch gekennzeichnet, dass $R_1$ und $R_2$ unabhängig voneinander Wasserstoff, $R_3$ $C_1$-$C_{12}$-Alkyl oder durch Fluor, Chlor, Brom, Dimethylamino, Methoxy, Aethoxy, Methylthio, Aethylthio, Cyclopropyl, Cyclopentyl, Cyclohexyl oder Phenyl substituiertes $C_1$-$C_{12}$-Alkyl bedeuten, wobei der Phenylrest jeweils durch Fluor, Chlor, Brom, Methyl, Methoxy, Trifluormethyl, Trifluormethoxy oder Nitro substituiert sein kann.

8. Verbindungen gemäss Anspruch 1 ausgewählt aus der Reihe
4-Chlor-4,4-difluorthiobuttersäure-S-benzylester,
4-Chlor-4,4-difluorthiobuttersäure-S-phenylester,
4-Chlor-4,4-difluorthiobuttersäure-S-(4-toluyl)-ester und
4-Chlor-4,4-difluorthiobuttersäure-(3-chlor-phenyl)-ester.

9. Verfahren zur Herstellung der Verbindungen der Formel I gemäss Anspruch 1, dadurch gekennzeichnet, dass man entweder
a) ein 4-Chlor-4,4-difluorbuttersäurehalogenid der Formel II

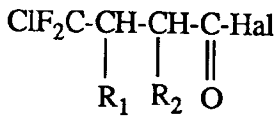

(II)

worin $R_1$ und $R_2$ die unter Formel I gegebenen Bedeutungen haben und Hal für Halogen, vorzugsweise Chlor oder Brom steht, vorzugsweise in Gegenwart einer Base mit einem Mercaptan der Formel III

$$H\text{-}S\text{-}R_3 \qquad (III)$$

worin $R_3$ die unter Formel I gegebene Bedeutung hat, umsetzt, oder
b) eine 4-Chlor-4,4-difluorbuttersäure der Formel IV

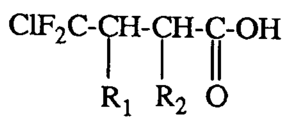

(IV)

worin $R_1$ und $R_2$ die unter Formel I gegebenen Bedeutungen haben, in Gegenwart eines wasserabspaltenden Mittels mit einem Mercaptan der Formel III umsetzt.

10. Schädlingsbekämpfungsmittel, welches als aktive Komponente mindestens eine Verbindung der Formel I nach Anspruch 1 enthält.

11. Mittel gemäss Anspruch 10, dadurch gekennzeichnet, dass es zusätzlich noch mindestens einen Träger enthält.

12. Verwendung einer Verbindung der Formel I nach Anspruch 1 zur Bekämpfung von Schädlingen an Tieren und Pflanzen.

13. Verwendung gemäss Anspruch 12, dadurch gekennzeichnet, dass es sich bei den Schädlingen um pflanzenschädigende Insekten und Arachniden handelt.

14. Verfahren zur Bekämpfung von tier- und pflanzenschädigenden Insekten und Arachniden, dadurch gekennzeichnet, dass man die Schädlinge oder ihren Lebensraum mit einer wirksamen Menge einer Verbindung der Formel I gemäss Anspruch 1 behandelt.

**Patentansprüche für folgenden Vertragsstaat: ES**

1. Verfahren zur Herstellung der 4-Chlor-4,4-difluorthiobuttersäure-Derivate Formel I

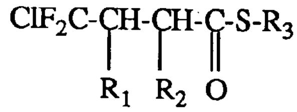

(I)

worin
$R_1$ und $R_2$ unabhängig voneinander Wasserstoff, $C_1$-$C_4$-Alkyl oder $C_1$-$C_4$-Halogenalkyl und
$R_3$ Wasserstoff oder einen organischen Rest bedeuten, dadurch gekennzeichnet, dass man entweder
a) ein 4-Chlor-4,4-difluorbuttersäurehalogenid der Formel II

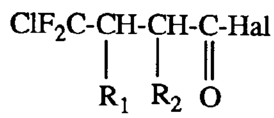

(II)

worin $R_1$ und $R_2$ die unter Formel I gegebenen Bedeutungen haben und Hal für Halogen, vorzugsweise Chlor oder Brom steht, vorzugsweise in Gegenwart einer Base mit einem Mercaptan der Formel III

$$H\text{-}S\text{-}R_3 \qquad (III)$$

worin $R_3$ die unter Formel I gegebene Bedeutung hat, umsetzt, oder
b) eine 4-Chlor-4,4-difluorbuttersäure der Formel IV

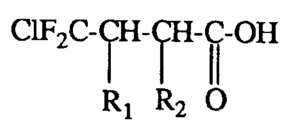

(IV)

worin $R_1$ und $R_2$ die unter Formel I gegebenen Bedeutungen haben, in Gegenwart eines wasserabspaltenden Mittels mit einem Mercaptan der Formel III umsetzt.

2. Verfahren gemäss Anspruch 1, dadurch gekennzeichnet dass $R_3$ für Wasserstoff oder für jeweils substituiertes oder unsubstituiertes $C_1$-$C_{20}$-Alkyl, $C_3$-$C_6$-Cycloalkyl, $C_3$-$C_{10}$-Alkenyl, Benzyl oder Aryl steht.

3. Verfahren gemäss Anspruch 1, dadurch gekennzeichnet, dass $R_3$ Wasserstoff, $C_1$-$C_{20}$-Alkyl, $C_3$-$C_6$-Cycloalkyl, $C_3$-$C_{10}$-Alkenyl, Aryl, $C_3$-$C_{10}$-Halogenalkenyl; durch Halogen subsrituiertes $C_3$-$C_6$-Cycloalkyl; durch Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_6$-Alkoxycarbonyl, $C_1$-$C_4$-Alkylthio oder Nitro substituiertes Aryl; oder durch Halogen, Di-$C_1$-$C_4$-alkylamino, $C_1$-$C_4$-Alkoxy, $C_1$-$C_4$-Alkylthio, $C_1$-$C_{20}$-Alkoxycarbonyl, $C_3$-$C_7$Cycloalkoxycarbonyl, $C_1$-$C_4$-Alkylcarbonyloxy, $C_3$-$C_6$-Cycloalkyl, Aryl, $C_1$-$C_{10}$-Alkylaminocarbonyl oder Anilinocarbonyl substituiertes $C_1$-$C_{20}$-Alkyl steht; wobei die Arylgruppen jeweils durch Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder Nitro substituiert sein können.

4. Verfahren gemäss Anspruch 1, dadurch gekennzeichnet, dass $R_1$ und $R_2$ unabhängig voneinander Wasserstoff bedeuten.

5. Verfahren gemäss Anspruch 3, dadurch gekennzeichnet, dass $R_3$ für $C_1$-$C_{20}$-Alkyl, $C_3$-$C_6$-Cycloalkyl, $C_3$-$C_{10}$-Alkenyl, Phenyl, Naphthyl, $C_3$-$C_{10}$-Halogenalkenyl, durchFluor, Chlor oder Brom substituiertes $C_3$-$C_6$-Cycloalkyl,durch Fluor, Chlor, Brom, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_4$-Alkoxycarbonyl, $C_1$-$C_3$-Alkylthio oder Nitro substituiertes Phenyl, oder durch Fluor, Chlor, Brom, Di-$C_1$-$C_4$-alkylamino, $C_1$-$C_4$-Alkoxy, $C_1$-$C_4$-Alkylthio, $C_1$-$C_{10}$-Alkylcarbonyl, $C_1$-$C_{20}$-Alkoxycarbonyl, $C_3$-$C_7$-Cycloalkoxycarbonyl, $C_1$-$C_4$-Alkylcarbonyloxy, $C_3$-$C_6$-Cycloalkyl, Phenyl, $C_1$-$C_{10}$-Alkylaminocarbonyl oder Anilinocarbonyl substituiertes $C_1$-$C_{20}$-Alkyl steht, wobei die Phenylgruppen jeweils durch Fluor, Chlor, Brom, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio oder Nitro substituiert sein können.

6. Verfahren gemäss Anspruch 1, dadurch gekennzeichnet, dass $R_1$ und $R_2$ unabhängig voneinander Wasserstoff, $R_3$ Phenyl, Benzyl, oder jeweils durch Fluor, Chlor, Brom, $C_1$-$C_3$-Alkyl, $C_1$-$C_3$-Halogenalkyl, $C_1$-$C_3$-Alkoxy, $C_1$-$C_4$-Alkoxycarbonyl, $C_1$-$C_3$-Halogenalkoxy, $C_1$-$C_3$-Alkylthio oder Nitro, substituiertes Phenyl oder Benzyl bedeuten.

7. Verfahren gemäss Anspruch 1, dadurch gekennzeichnet, dass $R_1$ und $R_2$ unabhängig voneinander Wasserstoff, $R_3$ $C_1$-$C_{12}$-Alkyl oder durch Fluor, Chlor, Brom, Dimethylamino, Methoxy, Aethoxy, Methylthio, Aethylthio, Cyclopropyl, Cyclopentyl, Cyclohexyl oder Phenyl substituiertes $C_1$-$C_{12}$-Alkyl bedeuten, wobei der Phenylrest jeweils durch Fluor, Chlor, Brom, Methyl, Methoxy, Trifluormethyl, Trifluormethoxy oder Nitro substituiert sein kann.

8. Verfahren gemäss Anspruch 1 zur Herstellung eines Wirkstoffs der Formel I ausgewählt aus der Reihe
4-Chlor-4,4-difluorthiobuttersäure-S-benzylester,
4-Chlor-4,4-difluorthiobuttersäure-S-phenylester,
4-Chlor-4,4-difluorthiobuttersäure-S-(4-toluyl)-ester und
4-Chlor-4,4-difluorthiobuttersäure-(3-chlorphenyl)-ester.

9. Schädlingsbekämpfungsmittel, welches als aktive Komponente mindestens eine Verbindung der Formel I nach Anspruch 1 enthält.

10. Mittel gemäss Anspruch 9, dadurch gekennzeichnet, dass es zusätzlich noch mindestens einen Träger enthält.

11. Verwendung einer Verbindung der Formel I nach Anspruch 1 zur Bekämpfung von Schädlingen an Tieren und Pflanzen.

12. Verwendung gemäss Anspruch 11, dadurch gekennzeichnet, dass es sich bei den Schädlingen um pflanzenschädigende Insekten und Arachniden handelt.

13. Verfahren zur Bekämpfung von tier- und pflanzenschädigenden Insekten und Arachniden, dadurch gekennzeichnet, dass man die Schädlinge oder ihren Lebensraum mit einer wirksamen Menge einer Verbindung der Formel I gemäss Anspruch 1 behandelt.

Europäisches Patentamt

# EUROPÄISCHER RECHERCHENBERICHT

Nummer der Anmeldung

EP 91810640.2

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
| A | US - A - 3 324 163 (M. HAUPTSCHEIN et al.) * Gesamt * -- | 1,9 | C 07 C 327/22<br>A 01 N 37/02 |
| A | EP - A1 - 0 078 562 (S.E.R.E.S.C.I., S.P.R.L) * Ansprüche * ---- | 10-14 | |
| | | | RECHERCHIERTE SACHGEBIETE (Int. Cl.⁵) |
| | | | C 07 C 327/00<br>A 01 N 37/00 |

Der vorliegende Recherchenbericht wurde für alle Patentansprüche erstellt.

| Recherchenort | Abschlußdatum der Recherche | Prüfer |
|---|---|---|
| WIEN | 13-11-1991 | HOFBAUER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82